# EUROPÄISCHE PATENTSCHRIFT

(11) **EP 0 451 599 B1**
(45) Veröffentlichungstag und Bekanntmachung des Hinweises auf die Patenterteilung: **08.12.1993**
(21) Anmeldenummer: 91104725.6
(22) Anmeldetag: 26.03.1991
(51) Int. Cl.: C07C 275/40, C07C 273/18

(54) **Verfahren zur Herstellung von N'N-Bis(3-aminophenyl)-harnstoffen**
Process for the production of N'N-bis((3-aminophenyl)-ureas
Procédé pour la préparation de N'N-bis(amino-3 phényl)-urées

(30) Priorität: 07.04.1990 DE 4011360
(43) Veröffentlichungstag der Anmeldung: 16.10.1991
(73) Patentinhaber: BAYER AG, 51368 Leverkusen (DE)
(72) Erfinder: Ruckes, Andreas, Dr., W-5090 Leverkusen 3 (DE); Grögler, Gerhard, Dr., W-5090 Leverkusen (DE); Kopp, Richard, Dr., W-5000 Köln 80 (DE)

(56) Entgegenhaltungen:
- EP-A- 0 374 653
- US-A- 1 617 847
- US-A- 2 503 797

## Beschreibung

Die vorliegende Erfindung betrifft ein einfaches Verfahren zur Herstellung von N,N'-Bis(3-aminophenyl)harnstoffen der allgemeinen Formel (I), die gegebenenfalls geringe Mengen an oligomeren Harnstoffen der allgemeinen Struktur (II) enthalten können. 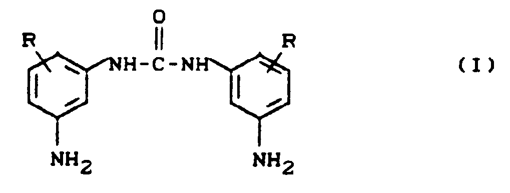 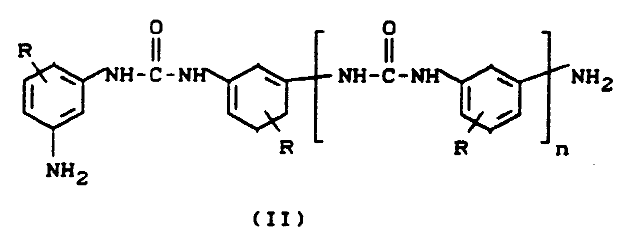
(R = lineare oder verzweigte Alkylgruppe mit 1 bis 6 C-Atomen, die in 2-, 4- und/oder 6-Stellung stehen können).

Das erfindungsgemäße Verfahren beinhaltet die Umsetzung von alkylsubstituierten m-Phenylendiaminen mit Harnstoff in einem Molverhältnis > 2:1 in Wasser als Lösungsmittel.

Die aus dem erfindungsgemäßen Verfahren erhaltenen Diaminodiphenylharnstoffe können in fester oder gelöster Form als Kettenverlängerer zur Herstellung von Polyurethanpolyharnstoff-Elastomeren bzw. reinen Polyharnstoff-Elastomeren verwendet werden. Die erfindungsgemäßen Diaminodiphenylharnstoffe können z.B. auch als Kopplungskomponente für Diazofarbstoffe, als Härter für Epoxid- und Phenolharze sowie für alle anderen an sich bekannten Reaktionen von Aminen wie Amid- oder Imidbildung und andere verwendet werden.

Es ist bekannt, Diaminodiphenylharnstoffe durch Phosgenierung entsprechender Nitroaniline zum Dinitrodiphenylharnstoff und anschließender katalytischer Reduktion zum Diamin zu erhalten, wie z.B. die von I.L. Khmel'nitskaya et al. im Zh. Obsh. Khim 30 (2) (1960) auf der S. 602 beschriebene Synthese von III oder die vom W.R. Turner und L.M. Werbel im J. Med. Chem. 28 (1985) auf der S. 1738 beschriebene Synthese des N,N'-Bis(5-amino-2-methylphenyl)-harnstoffes IV.

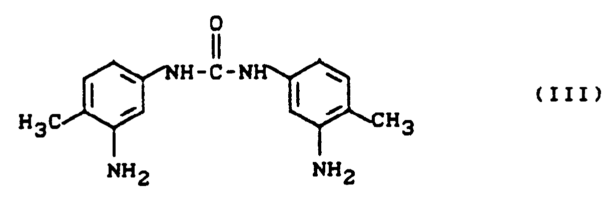

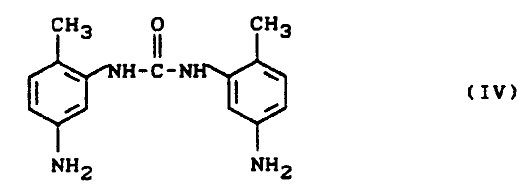

Nachteilig ist neben den i.a. schlechten Ausbeuten der Gesamtreaktion vor allem der kostenintensive Reduktionsschritt.

Eine weitere zweistufige Synthese zur Herstellung von Diaminodiphenylharnstoffen ist die von H. Schiff und A. Ostrogovick in Liebigs Ann., 293 1896 auf den Seiten 371 ff. am Beispiel des N,N'-Bis(4-aminophenyl)harnstoffes beschriebene Umsetzung von N-Acetyl-p-phenylendiamin mit Harnstoff und anschließender Verseifung der Acetyl-Schutzgruppe. Nachteilig hierbei ist, daß monoacetylierte Diamine, die in der Regel nicht einfach herstellbar sind, als Ausgangskomponenten verwendet werden müssen.

Spezielle p-Phenylendiamine, in denen die Reaktivität einer NH₂-Gruppe durch geeignete o-Substituenten stark erniedrigt ist, können mit Phosgen direkt zu den entsprechenden Aminocarbaniliden umgesetzt werden. Geeignete Diamine sind z.B. 2,5-Diaminosulfonsäure (DRP-140613, Frl. 11, 1292) oder 2,6-Dichlor-p-phenylendiamin (DRP-268658, Frl. 11, 164). Andere Phenylendiamine ergeben unter solchen Bedingungen praktisch ausschließlich Polyharnstoffe, die als reaktive Kettenverlängerer völlig ungeeignet sind.

Schließlich lehrt die US-PS 1 617 847 die Herstellung von N,N'-Bis(4-aminophenyl)harnstoffen durch Reaktion von p-Phenylendiamin und alkylsubstituierten p-Phenylendiaminen mit Harnstoff in Substanz oder in inerten Lösungsmitteln, wie z.B. o-Dichlorbenzol.

Zusammenfassend kann gesagt werden, daß alle bisher bekannten Verfahren entweder aufwendig sind, nur für spezielle Diamine geeignet sind oder nur mit p-substituierten Phenylendiaminen als Ausgangsverbindungen zu den gewünschten niedermolekularen Harnstoffen führen.

Aufgabe dieser Erfindung war es, eine einfache Synthese für Harnstoffe oder der Basis von ortho-alkylsubstituierten m-Phenylendiaminen zu finden.

Zwei Aspekte sind dabei besonders zu beachten. Auf der einen Seite sollte das Verfahren zu möglichst niedermolekularen Harnstoffen führen, da wie schon erwähnt Polyharnstoffe aufgrund zu geringer Reaktivität nicht mehr als Kettenverlängerer geeignet sind. Auf der anderen Seite sollte der Anteil an monomerem Ausgangsamin in den Harnstoffen so weit wie möglich reduziert sein, um jegliche physiologische Gefährdung beim Umgang mit dem Produkt sowie dem bekannten negativen Einfluß von freien, aromatischen, niedermolekularen Aminen auf die Licht- und Verfärbungsstabilität der damit hergestellten PUR-Kunststoffe auszuschalten.

Überraschenderweise gelang die Lösung dieser Aufgabe durch die Reaktion von ortho-alkylsubstituierten m-Phenylendiaminen mit Harnstoff in wasser als Lösungsmittel. Die Reaktion muß in einem bestimmten Phenylendiamin/Harnstoff-Molverhältnis >2:1 durchgeführt werden.

In der US-PS 2 503 797 ist zwar schon die Umsetzung von p-Phenylendiaminen mit Harnstoff in wäßriger Lösung beschrieben, es wird jedoch ausdrücklich darauf hingewiesen und auch mit Beispielen belegt, daß die entsprechenden m-Phenylendiamine nur dann oligomerenarm und in guter Ausbeute erhalten werden, wenn 4 Äquivalente Schwefelsäure zugesetzt werden. Zur Aufarbeitung muß das ausgefallene schwefelsaure Salz des Harnstoffs mit BaCl₂ in das entsprechende Chlorid umgewandelt werden, aus dem dann die freie Base erhalten werden kann.

Um so überraschender war es deshalb, daß unter den im folgende genau beschriebenen, speziellen Reaktionsbedingungen die Umsetzung von m-Phenylendiaminen mit Harnstoff in wäßriger Lösung zu oligomerenarmen Diaminodiphenylharnstoffen in guten bis sehr guten Ausbeuten gelingt.

Gegenstand der Erfindung ist somit ein Verfahren zur Herstellung von N,N'-Bis(3-aminophenyl)harnstoffen der allgemeinen Formel (I), worin 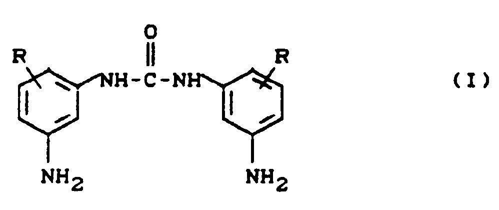
- R: eine lineare oder verzweigte Alkylgruppe mit 1 bis 6 Kohlenstoffatomen bedeutet, die sich in 2-, 4- und/oder 6-Stellung befinden kann, dadurch gekennzeichnet, daß die entsprechenden Phenylendiamine mit Harnstoff in einem Molverhältnis >2:1 in Wasser als Lösungsmittel umgesetzt werden.

Im folgenden werden die Verfahrensbedingungen genauer erläutert.

Besonders geeignet für das Verfahren sind 1-Alkyl-2,4-diaminobenzole, wie z.B. Toluylen-2,4-diamin. Die den Ansprüchen genügenden Verbindungen können entweder allein oder auch in Kombination miteinander eingesetzt werden.

Als Lösungsmittel wird Wasser verwendet.

m-Phenylendiamin und Harnstoff werden in einem Molverhältnis >2:1, bevorzugt >3:1 bis 10:1, besonders bevorzugt >3:1 bis 5:1 eingesetzt. Werden Molverhältnisse = (2:1 gewählt, erhält man Produkte mit zu niedrigen NH-Zahlen, d.h. stark vorverlängerte Harnstoffe. Im besonders bevorzugten Bereich werden praktisch Produkte mit NH-Zahlen nahe der Theorie erhalten. Erhöhung des Molverhältnisses ist prinzipiell möglich, führt aber auf der einen Seite zu keiner Verbesserung des Produktes und ist auf der anderen Seite auch wirtschaftlich nicht sinnvoll.

Die m-Phenylendiamine werden in Mengen zwischen 20 und 200 Gew.-Teilen, vorzugsweise zwischen 40 und 150 Gew.-Teilen und besonders bevorzugt zwischen 80 und 120 Gew.-Teilen, bezogen auf 100 Gew.-Teile Lösungsmittel, im Wasser gelöst. wird die Konzentration zu stark erhöht, so ist der Reaktionsansatz durch ausfallendes Produkt zunehmend schlechter rührbar und zusätzlich enthält das Produkt erhebliche Mengen an mitgerissenem Ausgangsamin, welches nur durch aufwendige Reinigungsoperationen entfernt werden kann. Im bevorzugten Konzentrationsbereich werden NH-Zahlen nahe der Theorie erhalten. Zu der Lösung des Amins im Wasser, wird der Harnstoff im beschriebenen Molverhältnis gegeben und die Reaktionsmischung bei Temperaturen zwischen 60-100°C, bevorzugt unter Rückfluß, gerührt.

Das ausgefallene Produkt wird abfiltriert und zum Entfernen restlicher Mengen Ausgangsamin mit Wasser gewaschen. Von Vorteil erweist sich hierbei, vor dem Filtrieren Wasser zuzufügen und die Filtration in einer mit Dampf beheizten Filternutsche durchzuführen, um ein Ausfallen des Restamin bei niedrigen Temperaturen zu verhindern. Ebenso ist es aus den gleichen Gründen vorteilhaft mit heißem Wasser nachzuwaschen. Das aus der Mutterlauge gewinnbare überschüssige Ausgangsamin kann ohne weitere Reinigung wiederverwendet werden.

Nach dem Trocknen erhält man den gewünschten Harnstoff als feinkristallinen Feststoff in ausgezeichneter Ausbeute und mit NH-Zahlen, abhängig von den gewählten Konzentrationsverhältnissen, von >270 mg KOH/g (th. 416 mg KOH/g), vorzugsweise von >360 mg KOH/g, wobei der Gehalt an freiem monomerem Ausgangsamin <1 Gew.-%, vorzugsweise <0,5 Gew.-% beträgt.

Die nachfolgenden Beispiele erläutern das erfindungsgemäße Verfahren, ohne es jedoch einzuschränken (%-Angaben bedeuten Gew.-%, soweit nicht anderes vermerkt wurde).

### Beispiel 1 Vergleichsbeispiel aus US-PS 2 503 797 (nicht erfindungsgemäß)

54 Gew.-Tle. 1,3-Diaminobenzol werden in 110 Vol.-Tln. 38,6-%iger Schwefelsäure unter Rühren gelöst. Die Temperatur der Lösung steigt dabei auf 65-70°C. Nach der Zugabe von 19 Gew.-Tln. Harnstoff wird die Reaktionsmischung für 32 h unter Rückfluß gekocht. Anschließend werden 120 Gew.-Tle. Wasser zugegeben. Das ausgefallene Produkt wird abfiltriert und mit 200 Gew.-Tln. kaltem Wasser gewschen. Das so erhaltene schwefelsaure Salz kann mit üblichen Methoden in die freie Base überführt werden. Es ist von Vorteil vor der Basenfreisetzung eine Umsalzung mit BaCl₂ durchzuführen.
Dem Vergleichsbeispiel sind keine Angaben über Ausbeute und analytische Daten beigefügt.

### Beispiel 2 erfindungsgemäß

366 g (3 Mol) Toluylendiamin-2,4 (TDA-2,4) und 60 g (1 Mol) Harnstoff (Molverhältnis TDA-2,4/Harnstoff 3:1) werden in 400 ml Wasser gelöst. Die Reaktionslösung wird 20 Stunden unter Rückfluß gerührt. Anschließend werden 1000 ml Wasser zugesetzt und der Ansatz weitere 30 Minuten unter Rückfluß gehalten. Nach dem Abfiltrieren über eine mit Wasserdampf beheizte Nutsche wird der Rückstand 2 mal mit je 300 ml heißem (90°C) Wasser gewaschen. Anschließend wird das Produkt im Vakuumtrockenschrank vom anhaftenden Wasser befreit.
- Ausbeute:: 213 g (79 %, bezogen auf den eingesetzen Harnstoff)
- NH-Zahl (HClO₄/Eisessig):: 369 mg KOH/g
- TDA-2,4-Restgehalt (HPLC):: 0,353 Gew.-%.

## Patentansprüche

1. Verfahren zur Herstellung von N,N'-Bis(3-aminophenyl)harnstoffen der allgemeinen Formel (I), worin 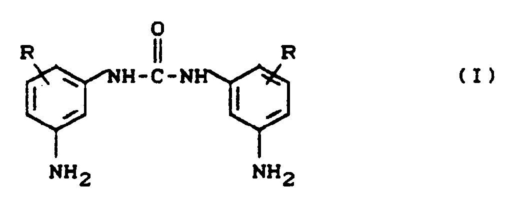
R eine lineare oder verzweigte Alkylgruppe mit 1 bis 6 Kohlenstoffatomen bedeutet, die sich in 2-, 4- und/oder 6-Stellung befinden kann, dadurch gekennzeichnet, daß die entsprechenden Phenylendiamine mit Harnstoff in einem Molverhältnis >2:1 in Wasser als Lösungsmittel umgesetzt werden.

2. Verfahren nach Anspruch 1, dadurch gekennzeichnet, daß das Molverhältnis von m-Phenylendiamin zu Harnstoff >3:1 beträgt.

3. Verfahren nach den Ansprüchen 1 und 2, dadurch gekennzeichnet, daß sich der Alkylsubstituent in 4-und/oder 6-Stellung befindet.

4. Verfahren nach den Ansprüchen 1 und 2, dadurch gekennzeichnet, daß der Alkylsubstituent eine Methyl-Gruppe ist.

## Claims

1. A process for the production of N,N'-bis-(3-aminophenyl)-ureas corresponding to general formula (I) 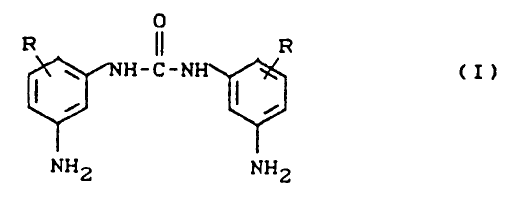 in which
R is a linear or branched alkyl group containing 1 to 6 carbon atoms which may be in the 2-, 4- and/or 6-position,
characterized in that the corresponding phenylenediamines are reacted with urea in a molar ratio of >2:1 in water as solvent.

2. A process as claimed in claim 1, characterized in that the molar ratio of m-phenylenediamine to urea is >3:1.

3. A process as claimed in claims 1 and 2, characterized in that the alkyl substituent is in the 4- and/or 6-position.

4. A process as claimed in claims 1 and 2, characterized in that the alkyl substituent is a methyl group.

## Revendications

1. Procédé de préparation de N,N'-bis-(3-aminophényl)-urées de formule générale I 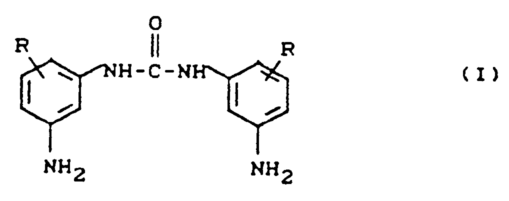 dans laquelle
R représente un groupe alkyle linéaire ou ramifié en C₁-C₆ qui peut se trouver en position 2, 4 et/ou 6, caractérisé en ce que l'on fait réagir les phénylènediamines correspondantes avec l'urée à un rapport molaire supérieur à 2:1 dans l'eau qui sert de solvant.

2. Procédé selon revendication 1, caractérisé en ce que le rapport molaire m-phénylènediamine/urée est supérieur à 3:1.

3. Procédé selon les revendications 1 et 2, caractérisé en ce que le substituant alkyle se trouve en position 4 et/ou 6.

4. Procédé selon les revendications 1 et 2, caractérisé en ce que le substituant alkyle est un groupe méthyle.
